(19) Europäisches Patentamt
European Patent Office
Office européen des brevets

(11) Publication number: 0 228 448 B1

# (12) EUROPEAN PATENT SPECIFICATION

(45) Date of publication of patent specification: 28.08.91 Bulletin 91/35

(51) Int. Cl.[5]: A61K 31/74

(21) Application number: 86904533.6

(22) Date of filing: 18.06.86

(86) International application number: PCT/US86/01315

(87) International publication number: WO 86/07539 31.12.86 Gazette 86/28

## (54) Use of biologically active copolymers for the manufacture of a medicament for stimulating the growth of an animal.

Divisional application 90102530.4 filed on 18/06/86.

(30) Priority: 18.06.85 US 745917
15.10.85 US 787770
13.06.86 US 872111

(43) Date of publication of application: 15.07.87 Bulletin 87/29

(45) Publication of the grant of the patent: 28.08.91 Bulletin 91/35

(84) Designated Contracting States: AT BE CH DE FR GB IT LI LU NL SE

(56) References cited:
US-A- 4 323 467

(56) References cited:
The Journal of Immunology, vol. 127, no. 3, September 1981, pages 1244-1250
Journal of the American Oil Chemists' Society, vol. 54, no. 3, 1977, pages 110-116
Federal Proceedings, vol. 44, March 1985, Article 7596 on page 1710
The Journal of Immunology, vol. 133, no. 6, December 1984, pages 3167-3175

(73) Proprietor: EMORY UNIVERSITY
1380 South Oxford Road
Atlanta, GA 30322 (US)

(72) Inventor: HUNTER, Robert, L.
3640 Churchwell Court
Tucker, GA 30084 (US)

(74) Representative: Sternagel, Hans-Günther, Dr. et al
Patentanwälte Dr. Michael Hann Dr. H.-G. Sternagel Sander Aue 30
W-5060 Bergisch Gladbach 2 (DE)

EP 0 228 448 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a further medical use of specific octablock copolymers and more particularly to the use of those compounds for the preparation of pharmaceutical compositions.

R. Hunter et al. describe in the Journal of Immunology, Vol. 133, No. 6, December 1984, pages 3167 to 3175 the adjuvant activity of nonionic octablock copolymer surfactants. The surfactants are block copolymers of hydrophilic polyoxyethylene and hydrophic polyoxypropylene units and having four active hydroxyl groups. The compounds produce a distinct pattern of immune response and inflammation when used in pharmaceutical compositions.

T.P. Atkinson et al. reported in Federal Proceedings, Vol. 44, March 1975, Article 7596, page 1710 on the induction of suppressor cells by the block copolymers described in Journal of Immunology, Vol 133. The suppression of antibody formation to immunogenic preparations of bovine serum albumin (BSA) is mentioned.

## Growth Promoting Compounds

With an ever-increasing world demand for food, there is constant pressure to increase the rate of production of food. In the early 1950s, researchers unexpectedly discovered that an antibiotic ingredient in chicken mash was a "growth factor." The finding drastically changed the nation's livestock and poultry production and was an economic boon for pharmaceutical companies. Feed animals are now raised under highly controlled conditions and receive specialized feed with a variety of growth promoting additives.

Routine antibiotic administration to animals has become almost universal since the discovery that the addition of small amounts of antibiotics such as penicillin, tetracycline and sulfamethazine, to animal feed increases the growth of pigs and cattle. In 1979, about 70% of the beef cattle and veal, 90% of the swine, and virtually 100% of broilers reared in the United States consumed antibiotics as part of their daily feed. This use, accounting for nearly 40% of antibiotics sold in the United States, is estimated to save consumers $3.5 billion a year in food costs.

Animals raised under modern conditions optimized for growth promotion receive rations containing high proportions of protein, usually in the form of soybean or cottonseed meal, and high percentages of grains such as corn or milo, a type of sorghum. Feed additives which have been used include such hormones as diethylstilbesterol, which also increases the rate of weight gain, and tranquilizers that prevent the effects of the stress brought on by confinement conditions from causing disease or weight loss.

Cattle ordinarily require 10 pounds of feed to produce one pound of weight gain. Under optimal growth promoting conditions and with enriched feed they gain one pound with only 6 pounds of feed.

Modern farming has greatly reduced the labor required to raise farm animals. In broiler chicken raising, where intensive methods have had the most dramatic effect, it took 16 hours of labor to raise a flock of 100 broilers in 1945; in 1970 that figure was reduced to 1.4 labor hours, in part because of the use of automated confinement facilities and associated advances in breeding and nutrition.

Although hormones and antibiotics have greatly increased the rate of growth of food animals, the use of such additives has not been without problems. One of the hormones that is commonly used as a growth stimulant, diethyl stilbesterol or DES, has been shown to be a carcinogen and has been banned from further use in most countries.

When antibiotics are mixed in animal feed, the compounds are spread throughout the environment exposing microorganisms to the antibiotics. The constant exposure of the microorganisms to antibiotics puts biological pressure on the microorganisms to develop a resistance to the antibiotics. This can result in a microorganism that is resistant to antibiotics and causes especially severe and difficult to treat infections.

An antibiotic-resistant microorganism is potentially a serious pathogen because it is difficult to control. If the organism causes an infection in an animal or in man, the infection may not be controlled with conventional antibiotics. If the infection is serious, there may not be time to determine which antibiotics are effective against the infecting bacteria. The problem has been especially serious when antibiotic resistant organisms in meat are consumed by people who themselves take antibiotics for treatment of disease. Antibiotics inhibit many of the normal microorganisms in the respiratory and gastrointestinal tracts. This allows the resistant ones to proliferate rapidly and produce more serious disease. The combination of antibiotic resistant organisms from food and ineffective antibiotic treatment of people has caused most of the deaths due to salmonella food poisoning reported in the Untied States in the past several years.

As a result of the increasing appearance of antibiotic resistant bacteria in feed lots and several serious epidemics caused by antibiotic resistant bacteria, there is increasing governmental pressure to ban the use of antibiotics in animal feed. Consequently, there is an immediate and increasing need for new, safe and effective growth stimulators of farm animals.

It is the object of the invention to provide a pharmaceutical composition comprising octablock copolymers for a further medical use.

This object is attained by the use of biologically active octablock copolymers having the following formula

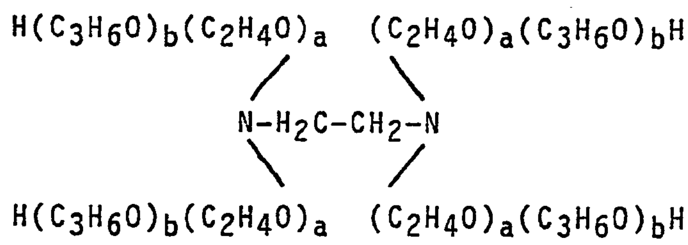

and

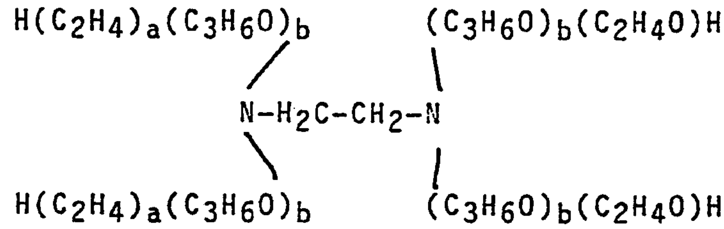

wherein:

the mean aggregrate molecular weight of the portion of the octablock copolymer represented by polyoxypropylene is between 5000 and 7000 daltons;

a is a number such that the portion represented by polyoxyethylene constitutes between 10 % to 40 % of the compound by weight; and

b is a number such that the polyoxypropylene portion of the total molecular weight of the octablock copolymer constitutes between 60 % and 90 % of the compound by weight for the preparation of a pharmaceutical composition for stimulating the growth of an animal.

The biologically active copolymers of the present invention are capable of stimulating the growth of an animal.

The biologically active copolymers have a wide variety of effects on individual cells. These compounds have ionophoric activity, *i.e.*, they cause certain ions to be transported across cell membranes. The compounds can cause non-cytolytic mast cell degranulation with subsequent histamine release.

The pharmaceutical compositions comprising the biologically-active copolymers of the present invention can be administered orally to animals.

The biologically-active copolymers can also be added to cattle feed to effect a change in the population of microorganisms normally resident in the rumen. Under normal conditions, the microorganisms digest the cellulose that is eaten by the cattle to the end-product methane. Methane is essentially unusable by the cattle. By administering the biologically-active copolymers of the present invention orally to the cattle, the copolymer differentially affects the rumen microorganism so that there is a shift in the rumen population of microorganisms resulting in an increase in proprionic acid production and a decrease in lactic acid and methane. Cattle are capable of using proprionate in their own metabolism thereby increasing the efficiency of food conversion.

Biologic effects of the copolymers vary with the structure of the polymer. The ability to modify the structure of these polymers to optimize particular biologic effects provides the potential to design synthetic compounds with a precision and ease not possible in other systems.

The biologically-active copolymer comprises a copolymer of polyoxyethylene (POE) which is hydrophilic and polyoxypropylene (POP) which is hydrophobic. The block copolymer is built on a tetrafunctional ethylenediamine initiator. In the preferred embodiment of the biologically-active copolymers, the block copolymers that comprise the biologically active copolymers have the following general formula:

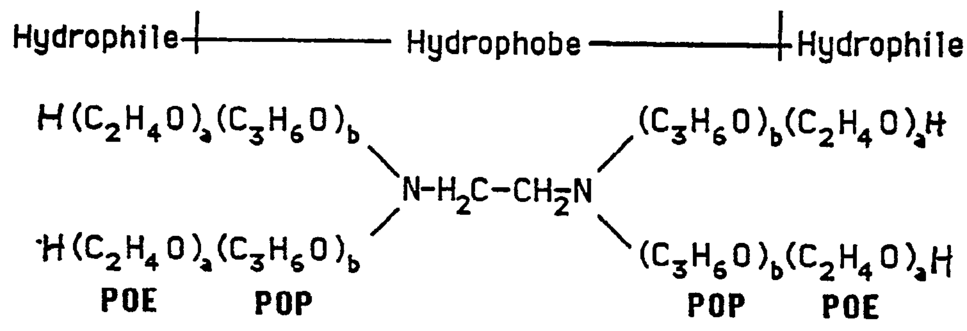

wherein:

the mean aggregate molecular weight of the hydrophobe portion of the octablock copolymer consisting of polyoxypropylene $(C_3H_6O)_b$ (POP) is between approximately 5000 and 7000 daltons;

a is a number such that the hydrophile portion represented by polyoxyethylene $(C_2H_4O)_a$ (POE) constitutes between approximately 10% and 40% of the total molecular weight of the compound; and

b is a number such that the polyoxypropylene $(C_3H_6O)_b$ (POP) portion of the total molecular weight of the octablock copolymer constitutes between 60% and 90% of the compound.

In another embodiment of the biologically-active copolymer, the block copolymer comprises a polymer of hydrophilic polyoxyethylene (POE) built on an ethylene diamine initiator. Polymers of hydrophobic polyoxypropylene (POP) are then added to block of hydrophilic polyoxyethylene (POE). This results in an octablock copolymer with the following general formula:

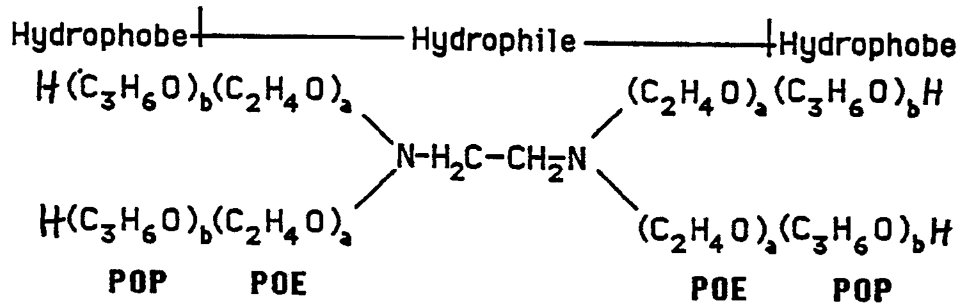

wherein:

a is a number such that the hydrophile portion represented by polyoxyethylene $(C_2H_4O)a$ (POE) constitutes between 10% to 40% of the total molecular weight of the compound;

the mean aggregate molecular weight of the hydrophobe portion of the octablock copolymer consisting of polyoxypropylene $(C_3H_6O)_b$ (POP) is between 5000 and 7000 daltons; and

b is a number such that the polyoxypropylene $(C_3H_6O)_b$ (POP) portion of the total molecular weight of the octablock copolymer constitutes between 60% and 90% of the compound. The composition comprising the biologically-active copolymer is usually administered by a subcutaneous, intravenous, or intramuscular injection of an effective amount of the copolymer into an animal or human. The biologically-active copolymer can be taken orally if it is desired that the copolymer have an effect on alimentary canal microorganisms. Normally, very little of the copolymer is absorbed from the alimentary canal.

The compositions prepared according to the present invention accelerate and prolong growth of an animal. The compositions prepared according to the invention are capable of altering the metabolism of ruminant animals so that the efficiency of feed conversion is increased and resulting in a stimulation of the growth.

These advantages of the present invention will become apparent after a review of the following detailed description of the disclosed embodiment and the appended claims.

The present invention uses a class of biologically active copolymers which have a wide variety of biological functions which are useful in treating various pathological conditions in both humans and animals and which can also be used to increase the efficiency of food production.

The biologically-active copolymers comprise a surface active compound with hydrophobic segments and a small proportion of hydrophile that is built upon an initiator compound. The initiator compound typically has

one or more active hydrogens upon which the hydrophobic or hydrophilic polymer units are condensed. Compounds that can be used as initiators in producing the biologically active copolymers of the present invention include methylamine, ethylamine, propylamine, butylamine, amylamine, hexylamine, aniline, the alkylene polyamines, especially aliphatic primary diamines such as ethylenediamine, propylene diamine, diethylenetriamine, triethylenetetramine, tetraethylenepentamine, hexamethylene-diamine, and phenylenediamine. Alkanolamines such as monoethanolamine, diethanolamine, triethanolamine, isopropanolamine, tri(t-propanol)amine, 2-amino-1-butanol, N-butyl-di(2-propanol)amine can also be used as initiators. Furthermore, heterocyclic compounds containing a hetero nitrogen atom can be employed, such as piperazine, 2 methylpiperazine, 2,5-dimethylpiperazine, imidazimidazole, pyrazolidine, pyrazolidone hydantoin, and dimethyl hydantoin. Hydroxyl amine and the hydroxylamine derivatives and aminophenol and aminophenol derivatives can also be used as initiators to produce the biologically active copolymers used for the present invention.

The compositions of this invention comprise surface active mixtures of conjugated polyoxypropylene-polyoxyethylene compounds based on the reactive hydrogen compounds wherein chains of oxypropylene groups having a defined molecular weight are attached to the initiator of the reactive hydrogen compound at the sites of the reactive hydrogen atoms and wherein chains of oxyethylene groups are then attached to the ends of the oxypropylene chains. Alternatively, the chains of oxyethylene groups having a defined molecular weight can be attached to the initiator of the reactive hydrogen compound at the sites of the reactive hydrogen atoms and then chains of oxypropylene groups can be attached to the ends of the oxyethylene chains.

The compounds for the compositions are prepared by condensing either ethylene oxide or propylene oxide with the reactive hydrogen on the initiator compound. After the first block of monomer unit have been added to the initiator molecule, a second block of either propylene oxide or ethylene oxide is condensed with the reactive hydrogen on the end of the first block. It is to be understood that butylene oxide can be substituted, either all or part, for propylene oxide in the biologically active copolymers of the present invention.

It is to be noted that it is not necessary to use pure propylene oxide in producing the oxypropylene chains of the biologically active copolymers. Small amounts, for example, up to 5 weight percent, of ethylene oxide can be included in the propylene oxide employed to prepare the hydrophobic reactive hydrogen compound-propylene oxide condensate. Likewise, the ethylene oxide condensed with the hydrophobic propylene oxide-reactive hydrogen compound condensate can also contain small amounts, such as up to about 5 weight percent, of propylene oxide.

It is further to be noted that when molecular weight is stated in this specification and claims, unless otherwise noted, there is meant the average theoretical hydrophobe molecular weight which equals the total of the grams of the propylene oxide employed per mole of reactive hydrogen compound. It is well recognized in the field of alkylene oxide chemistry that the polyoxyalkylene compositions one obtains by condensing an alkylene oxide with a reactive hydrogen compound are actually mixtures of compounds rather than a single molecular compound. The mixture contains closely related homologues wherein the statistical average number of oxyalkylene groups equals the number of moles of the alkylene oxide employed and the individual members in the mixture contain varying numbers of oxyalkylene groups. Thus, the compositions of this invention are "mixtures" of compounds which are defined by molecular weight of the polyoxypropylene chains and weight percent of oxyethylene groups.

The biologically-active copolymers comprise a surface active compound with four hydrophobic segments and a small proportion of hydrophile. Typical examples have eight segments or octablock structure with a core of either a hydrophobic or hydrophilic central structure and a hydrophilic or hydrophobic outer structure as shown in the following schematic structures.

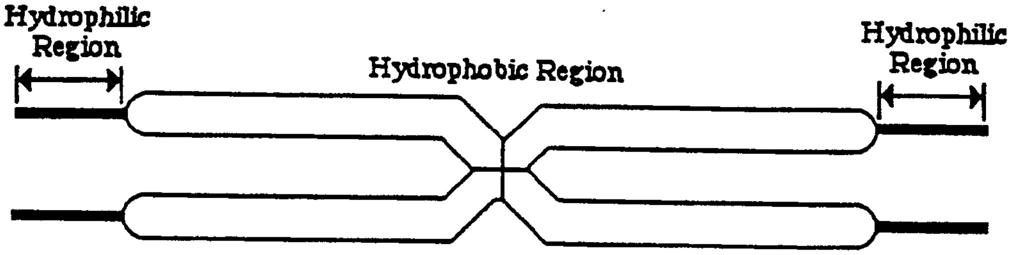


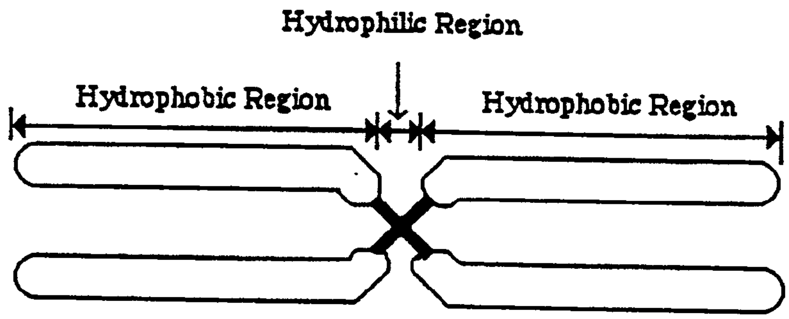


The entire molecule is poorly soluble in water and is either a nonionic or weakly cationic surface active agent. The steric configuration and physiochemical properties of the molecule, rather than the chemical nature of the constituent parts, are thought to be responsible for the biologic effects of the copolymer.

The biologically active compounds comprise blocks of polyoxypropylene and polyoxyethylene built on an alkylenediamine initiator. The blocks of polyoxypropylene (POP) and polyoxyethylene (POE) have the following structures:

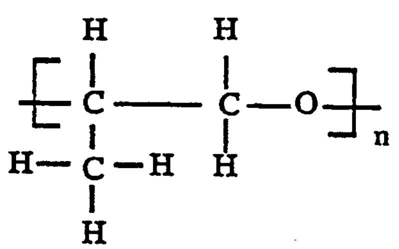
Polyoxypropylene (POP)

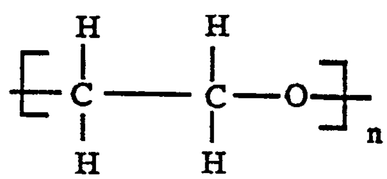
Polyoxyethylene (POE)

The polymer blocks are formed by condensation of ethylene oxide and propylene oxide onto a tetrafunctional ethylene diamine initiator at elevated temperature and pressure in the presence of a basic catalyst. There is some statistical variation in the number of monomer unit which combine to form a polymer chain in each copolymer. The molecular weights given are approximations of the average weight of copolymer molecule in each preparation. A further description of the preparation of these block copolymers is found in US-A-2,674,619 and US-A-2,979,528. (Also see "A Review of Block Polymer Surfactants", Schmolka, I.R., J. *Am. Oil Chemists' Soc.*, 54: 110-116 (1977) and *Block and Graft Copolymerization*, Volume 2 edited by R.J. Ceresa, John Wiley & Sons, New York, 1976)

In one embodiment of the biologically active copolymers of the present invention, the block copolymer comprises a polymer of hydrophobic polyoxypropylene (POP) built on an ethylenediamine initiator. Polymers of hydrophilic polyoxyethylene (POE) are then built on the block of hydrophobic polyoxypropylene (POP). This results in an octablock copolymer with the following general formula:

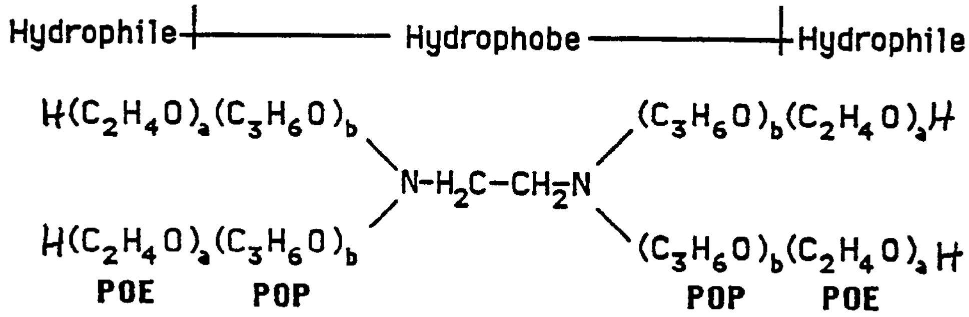

wherein:

the mean aggregate molecular weight of the hydrophobe portion of the octablock copolymer consisting of polyoxypropylene $(C_3H_6O)_b$ (POP) is between 5000 and 7000 daltons;

a is a number such that the hydrophile portion represented by polyoxyethylene $(C_2H_4O)_a$ (POE) constitutes between 10% to 40% of the total molecular weight of the compound;

b is a number such that the polyoxypropylene $(C_3H_6O)_b$ (POP) portion of the total molecular weight of the octablock copolymer constitutes between 60% and 90% of the compound; and

In another embodiment of the present invention, the block copolymer comprises a polymer of hydrophilic polyoxyethylene (POE) built on an ethylene diamine initiator. Polymers of hydrophobic polyoxypropylene (POP) are then built on the block of hydrophilic polyethylene (POE). This results in an octablock copolymer with the following general formula:

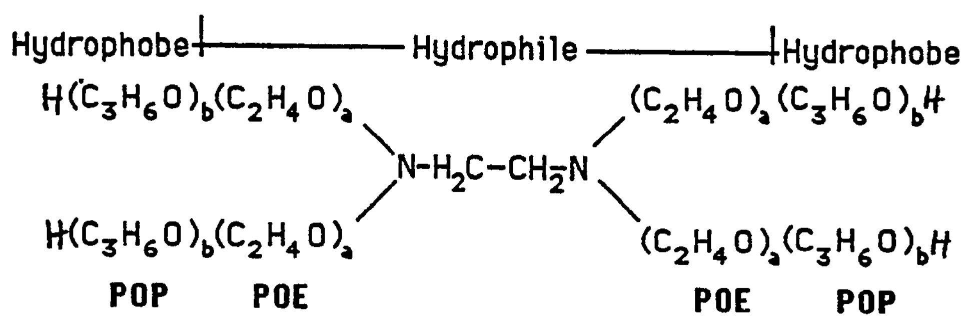

wherein:

The molecular weight of the hydrophobe portion of the octablock copolymer consisting of polyoxypropylene $(C_3H_6O)_b$ (POP) is between 5000 and 7000 daltons;

a is a number such that the hydrophile portion represented by polyoxyethylene $(C_2H_4O)_a$ (POE) constitutes between 10% and 40% of the total molecular weight of the compound;

b is a number such that the polyoxypropylene $(C_3H_6O)_b$ (POP) portion of the octablock copolymer constitutes between 60% and 90% of the compound; and

This type of polymer is called reverse copolymer because its structure is the reverse of octablock copolymers that have polyoxypropylene (POP) in the center flanked by blocks of polyoxyethylene (POE).

The octablock copolymers comprising the biologically active copolymers include, but are not limited to, the block copolymers Tetronic® and reverse Tetronic® manufactured by the BASF Corporation ((BASF Corporation, Parsippany, NJ).

A preferred biologically active copolymers is the octablock copolymer T130R2 (BASF Corporation, Parsippany, NJ) which corresponds to the following formula:

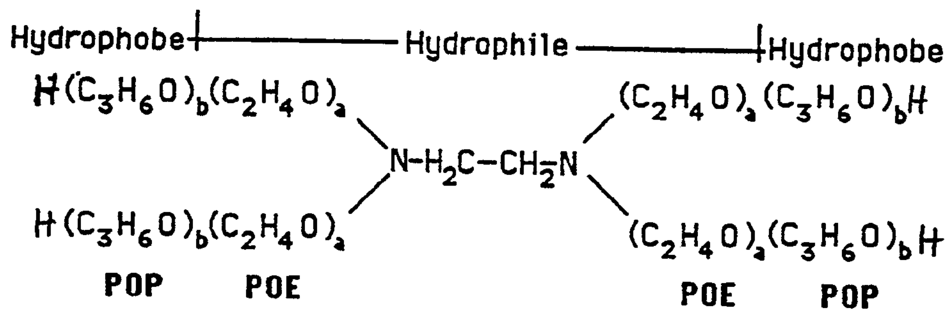

wherein:

The mean molecular weight of the hydrophobe portion of the octablock copolymer represented by polyoxypropylene $(C_3H_6O)_b$ (POP) is approximately 5750 daltons;

a is a number such that the hydrophile portion represented by polyoxyethylene $(C_2H_4O)_a$ (POE) constitutes 20% of the compound by weight; and

b is a number such that the polyoxypropylene $(C_3H_6O)_b$ (POP) portion of the octablock copolymer constitutes 80% of the compound by weight.

Another preferred embodiment of the biologically active copolymers is the compound designated T1501 (BASF Corporation, Parsippany, NJ) which corresponds to the following formula:

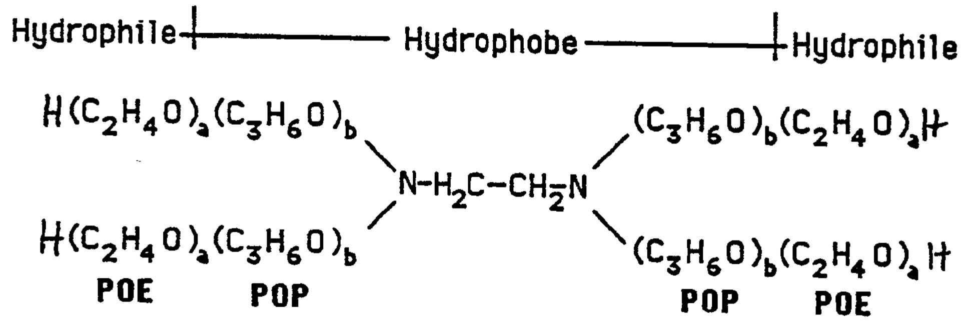

wherein:

the mean molecular weight of the hydrophobe portion of the octablock copolymer represented by polyoxypropylene $(C_3H_6O)_b$ (POP) is 6750 daltons;

a is a number such that the hydrophile portion represented by polyoxyethylene $(C_2H_4O)_a$ (POE) constitutes 10% of the compound by weight; and

b is a number such that the polyoxypropylene $(C_3H_6O)_b$ (POP) portion of the octablock copolymer constitutes 90% of the compound by weight.

The most preferred embodiment of the biologically active copolymers is the octablock copolymer T150R1 (BASF Corporation, Parsippany, NJ) which corresponds to the following formula:

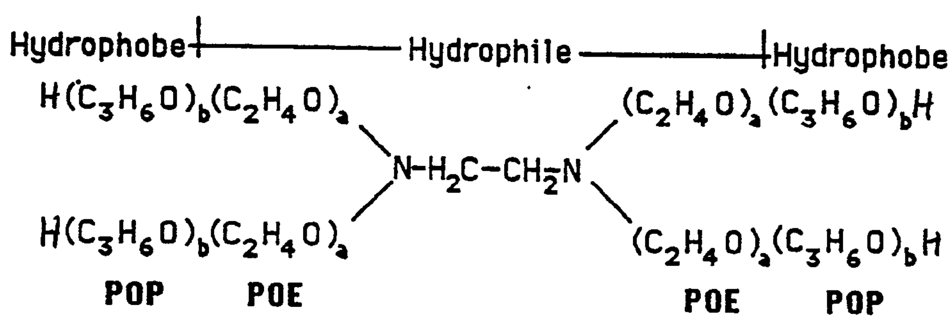

wherein:

The mean molecular weight of the hydrophobe portion of the octablock copolymer represented by polyoxypropylene $(C_3H_6O)_b$ (POP) is 6750 daltons;

a is a number such that the hydrophile portion represented by polyoxyethylene $(C_2H_4O)_a$ (POE) constitutes 10% of the compound by weight; and

b is a number such that the polyoxypropylene $(C_3H_6O)_b$ (POP) portion of the octablock copolymer constitutes 90% of the compound by weight.

Although not wanting to be bound by the following theory, it is believed that the biologically active polymers of the present invention act by the following mechanism:

Investigators have demonstrated hormone-mediated interactions between the thymus and the central nervous system. The biologically active copolymers are capable of affecting this relationship. It is believed that the present invention acts by mimicking the conformation and physicochemical properties of natural hormones and neuropeptides. The biologic responses induced by the biologically active copolymers of the present invention include:

1. Stimulation of rate and duration of growth of animals;

It is believed that there is a structural analogy between the biologically active copolymers and hormones, such as somatostatin, adrenocorticotropic hormone (ACTH), and β-endorphin. These peptides have in common a sequence of basic amino acids adjacent to spans of hydrophobic amino acids. These features are thought to be essential for function. Interaction of the oligoamine residues with cell surface anions and stabilization by adjacent hydrophobic moieties initiates the molecular program resulting in effector functions of the peptide. The block polymers have the same structural features: a central basic ethylenediamine flanked by spans of hydrophobic polyoxypropylene (POP). By means of inhibition with monovalent amines, we have evidence that the central ethylenediamine groups function like the basic moieties of peptide hormones in stimulating release of histamine from mast cells.

*In vitro* studies provide direct evidence that the biologically active copolymers of the present invention act in a manner analogous to somatostatin and ACTH. Mast cells have been used as a model to study receptor mediated mechanisms through which these hormones induce their biological receptor mediated mechanisms. Minute quantities of the polymers cause histamine release from mast cells by a temperature-dependent process which requires energy and calcium and can be blocked by specific inhibitors in a manner very similar to that of somatostatin and ACTH.

Both agonistic and antagonistic effect of hormones may be elicited by the polymers. Biologic effects of the polymers vary with the structure of the polymer. The ability to modify the structure of these polymers to optimize particular biologic effects provides potential to design synthetic drubs with a precision and ease not possible in other systems.

Many chelating agents, such as ethylene-diaminetetraacetic acid (EDTA) consist of oliboamine sites flanked by hydrogen bonding groups. The addition of flanking hydrophobic moieties produces ionophores which are able to transport ions across lipid containing membranes. The compounds have this general structure and can act as ionophores transporting cations across artificial membranes. Consequently, the compounds represent a new chemical type of ionophore. Some neuropeptide hormones (*e.g.*, substance P) have ionophore activity.

The biologically-active copolymers are generally poorly soluble in water. Although the compounds can be injected into an animal or human in aqueous media, it is preferable that the biologically-active copolymers used for the compositions of the present invention be injected as an oil-in-water or water-in-oil emulsion. A mineral oil or other oily substance such as Drakeol® 6VR or Drakeol® 5 (Penreco, Butler, PA) can be used as the oil phase of the emulsion. The aqueous phase can be physiologic phosphate buffered saline or other physiologic salt solution. The ratio of oil to water is preferably between 80:20 and 1:100.

Typically, an oil-in-water emulsion is prepared by mixing between approximately 0.5 to 50 grams of the biologically active copolymers with 5.0 ml of mineral oil in a Potter-Elvehjim homogenizer. Next, 95.0 ml of phosphate buffered saline (0.85 M sodium chlorid, pH 7.3) containing 0.2% polyoxyethylene sorbitan monooleate (Tween® 80, Atlas Chemical Industries, Wilmington, DE) and 50 mg bovine serum albumin (BSA, Sigma Chemical Co., St. Louis, MO) is added. The mixture homogenized thoroughly to form a fine emulsion. The BSA and Tween® 80 are used to stabilize the emulsion. It is to be understood that the method of preparing the emulsion, the proportions of oil and water and the type of oil used are not critical. An effective emulsion could be prepared by using a blender, by sonication or other means well known to those of ordinary skill in the art. It is to be further understood that other carriers, emulsifiers, aqueous solutions and adjuvants that are known to those of ordinary skill in the art can be used with the biologically active copolymers of the present invention.

Water-in-oil emulsions are prepared as follows. A stock oil-emulsifier mixture is prepared by blending mineral oil with approximately 5% to 10% of a water-in-oil emulsifier. A mixture of 94.5% oil (Drakeol®, Penreco, Butler, PA), 4.5% Sorbitan monooleate (Span® 80, Atlas Chemical Industries, Wilmington, DE) and 0.5%

polyoxyethylene sorbitan monooleate (Tween® 80, Atlas Chemical Industries, Wilmington, DE) is commonly used. A commercial blend, Freund's incomplete adjuvant, (Difco, Detroit, MI or Sigma Chemical, St. Louis, MO) is also suitable. Approximately 0.5 to 5.0 grams of the biologically active copolymers is added to either 60 ml of the oil-emulsifier mixture or to 40 ml of a physiologic saline solution similar to that used in the oil-in-water emulsion described above. The oil-emulsifier mixture is placed in a blender. The physiologic salt solution is added in three aliquots with vigorous homogenization to insure that a fine water-in-oil emulsion is prepared. Again, it is to be understood that the method of preparing the emulsion is not critical. Numerous variations of the composition of the aqueous and oil phases, their proportions and means of emulsification will be apparent to those skilled in the art and could be used with biologically active copolymers in practicing the invention.

The biologically active copolymers are effective with only one injection of compound being administered to an animal. However, in certain cases, subsequent injections may be necessary to achieve maximum stimulation.

The mode of injection can be subcutaneous, intramuscular or intravenous. A preferred mode of injection is subcutaneous. Intravenous injection is hazardous because of the toxic effect of embolic emulsions.

The optimum amount of the composition comprising biologically active copolymers in an injection varies with the size of the animal being treated. With animals such as rat or mice, the optimum amount of biologically active copolymers is between 0.5 and 5 mg per animal. With larger animals a larger quantity of biologically active copolymers is required in the injection for optimum results. With humans, cattle or swine, the dose varies with the age, size and condition of the individual but approximates 5 to 500 mg in most cases.

The following specific examples will illustrate the invention as it applies in particular to stimulating the growth in mice.

**Growth Stimulation, Comparison of Copolymers**

The biologically active copolymer was administered to mice. The growth of the mice was then measured after six weeks.

Oil-in-water emulsions were prepared with 1 mg bovine serum albumin (BSA), 50 mg of the indicated copolymer (all copolymers were manufactured by BASF Corporation, Parsipanny, NJ) and 100 µl mineral oil (Drake® 6VR, Penreco Refining Company, Butler, PA) in 2 ml of phosphate-buffered saline (PBS) with 0.2% Tween® 80 (Sigma Chemical Co., St. Louis, MO.). The mixture was homogenized in a Potter Elvehjim homogenizer. The oil, copolymer and BSA were homogenized together before adding the phosphate buffered saline. The mixture was then further homogenized to form a fine emulsion.

Emulsions were prepared of each of the copolymers shown in Table D. Each of these copolymers is comprised of blocks of polyoxyethylene (POE) and polyoxypropylene (POP) arranged in the fashion previously described for copolymer T150R1. They differ from one another only in the size of the blocks. Each has characteristic physiochemical and biologic properties.

Mice were injected with 50 µg of BSA, 2.5 mg copolymer and 5 mg oil in 0.1 ml of an oil-in-water emulsion divided between the two hind feet. Their weights six weeks later are summarized in Table E.

**Table E**

| Copolymer | Molecular Wt of Hydrophobe | Percent Hydrophile[a] | Mean Weight[b] |
|---|---|---|---|
| T90R1 | 3750 | 10% | 22.7 ± 1.03 grams |
| T110R1 | 4750 | 10% | 22.7 ± 0.76 grams |
| T130R1 | 5750 | 10% | 23.3 ± 1.36 grams |
| T130R2 | 5750 | 20% | 24.7 ± 0.62 grams |
| T150R1 | 6750 | 10% | 26.7 ± 1.75 grams |
| T150R4 | 6750 | 40% | 23.5 ± 1.77 grams |
| T150R8 | 6750 | 80% | 23.0 ± 1.06 grams |

a Percentages are to the nearest 10%
b Mean weight of groups of five mice ± standard deviation

The mice injected with the emulsion containing the copolymer T150R1 were visibly larger than those injected with any of the other copolymers. All of the mice appeared healthy.

All of the copolymers used in Table E consist of blocks of polyoxypropylene (POP) and polyoxyethylene (POE) attached in the same reverse octablock configuration The compounds differ from one another only in the size of the blocks of each compound. These differences, however, confer distinct physicochemical properties on the copolymers which correlate with their biologic activities.

**Growth Stimulation, Effect of Various Emulsions**

Oil-in-water emulsions were prepared using 1 mg bovine serum albumin (BSA), 50 mg of either copolymer T1501 or T150R1 (BASF Corporation, Parsippany, NJ) and 100 µl mineral oil (Drakeol® 6VR, Penreco, Butler, PA) in 2.0 ml phosphate buffered saline with 0.2% Tween® 80 in a Potter Evehjin homogenizer as described above. The control oil-in-water emulsion was prepared identically except that no copolymer was used.

Copolymer T1501 has the following general structure:

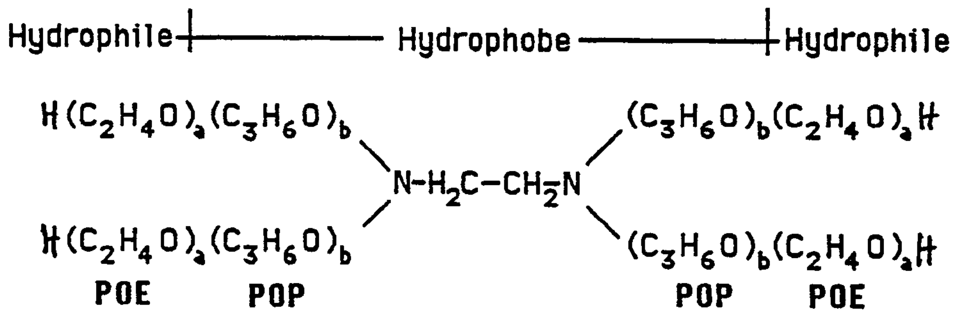

and copolymer T150R1 has the following general structure:

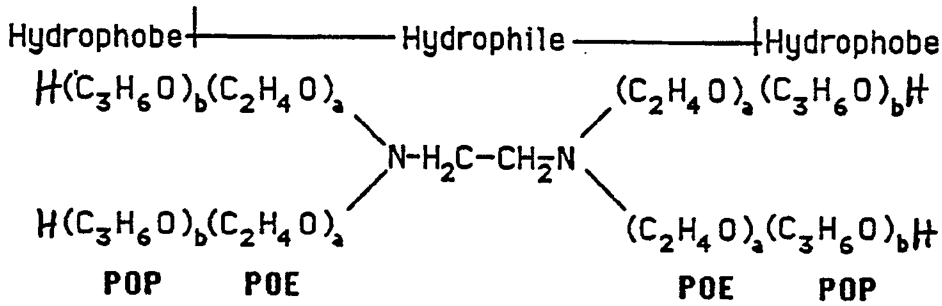


The hydrophobic polyoxypropylene block in each of the structures have a mean aggregate molecular weight of 6750. They each contain approximately 10% hydrophilic polyoxyehylene.

The water-in-oil emulsion was prepared using 50 mg of either copolymer T1501 or T150R1, 1.2 ml Freund's incomplete adjuvant (Sigma Chemical Company, St. Louis, MO), and 0.8 ml Hank's balanced salt solution, (Gibco, Grand Island, N.Y.), containing 1 mg bovine serum albumin (BSA). The control emulsion was prepared identically except that it contained no copolymer.

Mice were injected with 2.5 mg of copolymer in 0.1 ml of either the oil-in-water or water-in-oil emulsion. Controls consisted of mice injected with the same dose of an identical emulsion which contained no copolymer. The injections were given subcutaneously and the total dose was divided between the two hind feet. Their weights at six weeks are summarized in Table F.

## Table F

| COPOLYMER | MEAN WEIGHT[a] Oil-in-water Emulsion | MEAN WEIGHT[a] Water-in-Oil Emulsion |
|---|---|---|
| T1501 | 21.2 ± 1.92 grams | 26.6 ± 1.18 grams |
| T150R1 | 28.1 ± 0.72 grams | 26.5 ± 2.32 grams |
| Control | 22.5 ± 0.96 grams | 23.0 ± 1.41 grams |

a Mean weight of groups of five mice ± standard deviation

The block copolymer designated T150R1 was highly effective in stimulating growth in both water-in-oil emulsion and in oil-in-water emulsion. The block copolymer designated T1501 was effective in stimulating growth in a water-in-oil emulsion.

**Claims**

1. Use of biologically active octablock copolymers having the following formula

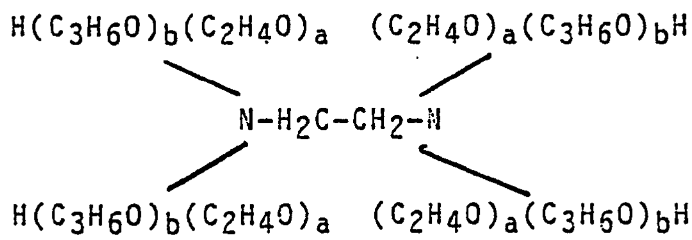

and

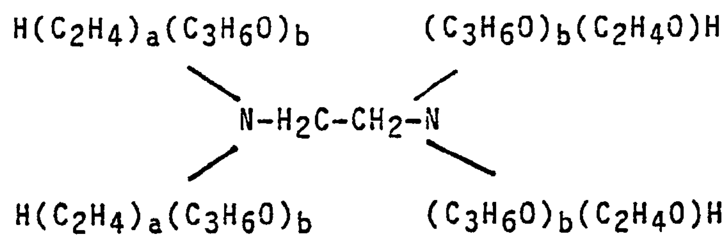

wherein:

the mean aggregrate molecular weight of the portion of the octablock copolymer represented by polyoxypropylene is between 5000 and 7000 daltons;

a is a number such that the portion represented by polyoxyethylene constitutes between 10 % to 40 % of the compound by weight; and

b is a number such that the polyoxypropylene portion of the total molecular weight of the octablock copolymer constitutes between 60 % and 90 % of the compound by weight for the preparation of a pharmaceutical composition for stimulating the growth of an animal.

2. Use of claim 1, wherein said octablock copolymer has the following structure:

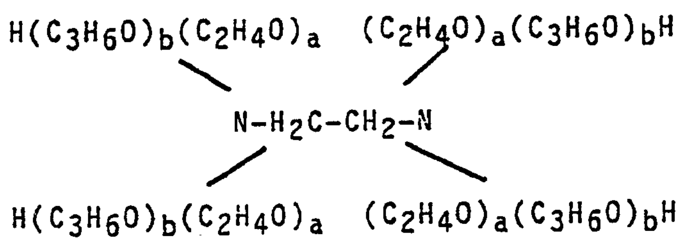

wherein

the mean aggregrate molecular weight of the portion of the octablock copolymer represented by polyoxypropylene is approximately 6750 daltons;

a is a number such that the portion of the total molecular weight represented by polyoxyethylene constitutes approximately 10 % of the compound by weight; and

b is a number such that the polyoxypropylene portion of the octablock copolymer constitutes approximately 90 % of the compound by weight.

**Patentansprüche**

1. Verwendung von biologisch aktiven Octablockcopolymeren der folgenden Formeln

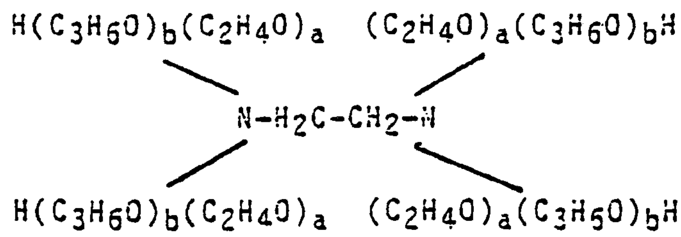

und

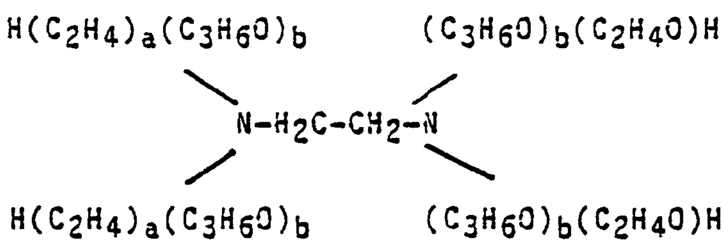

in denen

das aggregierte mittlere Molekulargewicht des Teiles des Octablockcopolymeren, das Polyoxypropylen ist, zwischen 5.000 und 7.000 Dalton beträgt,

a eine solche Zahl ist, daß der Anteil des Polyoxyethylens zwischen 10 % bis 40 % des Gewichts der Verbindung beträgt, und

b eine solche Zahl ist, daß der Polyoxypropylenanteil am Gesamtmolekulargewicht des Octablockcopolymeren zwischen 60 % und 90 % des Gewichts der Verbindung beträgt, zur Herstellung einer pharmazeutischen Zusammensetzung zur Anregung des Wachstums eines Tieres.

2. Verwendung nach Anspruch 1, **dadurch gekennzeichnet**, daß das Octablockcopolymer der Strukturformel genügt

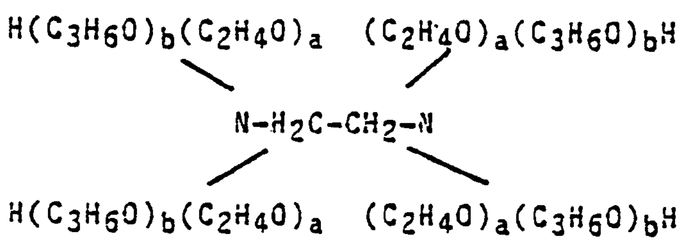

in der

das aggregierte mittlere Molekulargewicht des Teiles des Octablockcopolymeren, das Polyoxypropylen ist, etwa 6.750 Dalton beträgt,

a eine solche Zahl ist, daß der Anteil des Polyoxyethylens am Gesamtmolekulargewicht annäherend 10 % des Gewichts der Verbindung beträgt, und

b eine solche Zahl ist, daß der Polyoxypropylenanteil des Octablockcopolymeren etwa 90 % des Gewichts der Verbindung beträgt.

## Revendications

1. Utilisation de copolymères octablocs actifs sur le plan biologique ayant la formule suivante :

$H(C_3-H_6O)_b(C_2H_4O)_a$ $(C_2H_4O)_a(C_3H_6O)_bH$

\ /

$N-H_2C-CH_2-N$

/ \

$H(C_3H_6O)_b(C_2H_4O)_a$ $(C_2H_4O)_a(C_3H_6O)_bH$

et

$H(C_2H_4)_a(C_3H_6O)_b$ $(C_3H_6O)_b(C_2H_4O)H$

\ /

$N-H_2C-CH_2-N$

/ \

$H(C_2H_4)_a(C_3H_6O)_b$ $(C_3H_6O)_b(C_2H_4O)H$

dans laquelle

le poids moléculaire moyen global de la portion de copolymère octabloc représenté par le polyoxyéthylène est compris entre 5000 et 7000 daltons:

"a" est un nombre tel que la part représentée par le polyoxyéthylène constitue un pourcentage entre 10 et 40 % du composé en poids ; et

"b" est un nombre tel que la portion de polyoxypropylène du poids moléculaire total du copolymère octabloc constitue un pourcentage entre 60 et 90 % du composé en poids dans la préparation d'une composition pharmaceutique destinée à stimuler la croissance animale.

2. Utilisation du copolymère suivant la revendication 1 dans laquelle le copolymère octabloc mentionné a la structure suivante :

$H(C_3H_6O)_b(C_2H_4O)_a$ $(C_2H_4O)_a(C_3H_6O)_bH$

\ /

$N-H_2C-CH_2-N$

/ \

$H(C_3H_6O)_b(C_2H_4O)_a$ $(C_2H_4O)_a(C_3H_6O)_bH$

dans laquelle :

le poids moléculaire moyen global de la portion du copolymère octabloc représenté par le polyoxypropylène est approximativement de 6750 daltons ;

"a" est un nombre tel que la portion de poids moléculaire total représenté par le polyxypropylène constitue approximativement 10 % de composé en poids et

"b" est un nombre tel que la portion de polyoxypropylène du copolymère octabloc constitue approximativement 90 % du composé en poids.